# EUROPÄISCHE PATENTANMELDUNG

(11) **EP 4 085 854 A1**
(43) Veröffentlichungstag der Anmeldung: **09.11.2022**
(21) Anmeldenummer: 22181579.8
(22) Anmeldetag: 11.03.2016
(51) Int. Cl.: A61B 17/34, A61M 25/00, A61M 25/01, A61M 29/00, A61B 17/00

(54) **KANÜLENANORDNUNG**

(30) Priorität: 11.03.2015 AT 501972015; 06.11.2015 AT 509442015
(62) Teilanmeldung aus: 16159901.4
(71) Anmelder: Klepetko, Walter, 1180 Wien (AT)
(72) Erfinder: Klepetko, Walter, 1180 Wien (AT)
(74) Vertreter: Graf von Stosch Patentanwaltsgesellschaft mbH

(57) **Zusammenfassung**

Die Erfindung betrifft eine Kanülenanordnung zum Applizieren einer Strömungskanüle zum Ein-, Durch- oder Umleiten von Strömungsmedien, insbesondere Blut, im menschlichen oder tierischen Körper, wobei die Strömungskanüle (10) einen Hauptabschnitt (11) und einen Spitzenabschnitt (12) aufweist, dadurch gekennzeichnet, dass der Spitzenabschnitt (12) elastisch und in Funktionslage bogenförmig in einem Winkel vom Hauptabschnitt (11) abstehend ausgebildet ist, und dass der Spitzenabschnitt (12) in Applikationslage durch eine Führungsvorrichtung (14) in Richtung der Längserstreckung des Hauptabschnittes (11) gehalten ist. Weiters betrifft die Erfindung Anwendungen der Kanülenanordnung.

## Beschreibung

Die Erfindung betrifft eine Kanülenanordnung zum Applizieren einer Strömungskanüle zum Ein-, Durch- oder Umleiten von Strömungsmedien, insbesondere von Blut im menschlichen oder tierischen Körper, wobei die Strömungskanüle einen Hauptabschnitt und einen Spitzenabschnitt aufweist.

Weiters betrifft die Erfindung ein Verfahren zum Setzen von Kanülen und zur extrakorporalen Unterstützung von Patienten mit pulmonaler Hypertonie.

Kanülenanordnungen und Kathetersysteme sind seit langer Zeit in vielfältiger Form bekannt. So ist es auch bereits bekannt, Kanülen ausgehend von einem Einschnitt in der Leistengegend über die untere Hohlvene bis zum rechten Vorhof des Herzens zu legen. Es ist auch bekannt, über eine derartige Katheteranordnung mit einem Punktionsdraht und dem vorderen Ende des Katheters das Septum des Herzens zu durchstoßen und somit die Kanüle zum linken Vorhof des Herzens zu legen. Die transseptale Kanülierung des linken Vorhofs erweist sich als notwendig oder vorteilhaft zur temporären Behandlung schwerer pulmonaler Hypertension, sowie zur Anwendung bei konventionellen ECMO-Fällen (Extracorporeal membrane oxygenation) mit starken Sättigungsproblemen.

Schwerer Lungenhochdruck im Endstadium kann nur durch eine Transplantation der Lunge behandelt werden. Die Wartezeit auf ein passendes Spendeorgan kann jedoch in extremen Fällen nur durch eine mechanische Entlastung der pulmonalen Zirkulation überbrückt werden. Die derzeit dafür etablierte Methode stellt eine periphere venoarterielle ECMO dar, die jedoch ein beträchtliches Risiko für den Patienten darstellt.

Mit der vorliegenden Kanülenanordnung und deren Applikation gelingt es, durch interventionelle transseptale Kanülierung des linken Vorhofs und zusätzlich der Pulmonalarterie einen Umgehungskreislauf für die PH-Strombahn (PH= Pulmonale Hypertonie) herzustellen, wodurch es ermöglicht wird, den Patienten über längere Zeit zu stabilisieren. Bei dem vorgenannten Krankheitsbild kann die Vorgangsweise folgende Schritte umfassen:
- transseptale Kanülierung des linken Vorhofs (arteriell return),
- transtricuspidale Kanülierung der Pulmonalarterie (venus drainage) oder
- normale venöse Kanülierung,
- Konnektierung der Kanülen mit einer herkömmlichen Novalung ^{®} (bei transtricuspidaler Kanülierung) oder mit ILA-active (bei venöser Kanülierung) oder mit vergleichbaren Geräten.

Immer dann, wenn Kanülen Gewebeabschnitte durchstoße müssen, wie dies z.B.: beim Septum für die Kanülierung des linken Vorhofs notwendig ist, besteht das Problem, dass die Kanüle den für das Durchstoßen notwendigen Punktionsdraht mit dessen Punktionsspitze möglichst unter einem 90-grädigen Winkel auf den Gewebeabschnitt richten soll. Dies ist beim Septum des Herzens ein geringeres Problem, wenn die Kanülierung femoral erfolgt, also über die untere Hauptvene, da dann die in den linken Vorhof geführte Kanüle und der in ihr geführte Punktionsdraht annähernd mit 90°-igem Winkel an das Septum zu liegen kommt. Bei den derzeit bekannten Kanülenanordnungen ist es aber nicht möglich, den bevorzugten Zugang von der oberen Körperhälfte zu schaffen. Der Zugang von oben, beispielsweise über die obere Hohlvene, cervical oder subklavikulär, ist für den Patienten wesentlich angenehmer. Der Zugang hat eine bessere Dauerhaftigkeit und geringere Problemanfälligkeit. Weiters soll die Kanülenanordnung eine Verankerung im Septum zur Verhinderung von Dislokation aufweisen können. Die letztendlich erzielte Durchflussmenge muss ausreichend groß sein. Überdies ist es wünschenswert, die Kanüle und auch die sonstigen Teile der Kanülenanordnung mit antithrombotischen Eigenschaften zu versehen. Bevorzugt soll die Anordnung auch die Möglichkeit des Defektverschlusses im atrialen Septum bei eventueller Entfernung bieten. Und letztendlich soll die Kanülenanordnung auch die Konnektierung mit dem Novalung ^{®} - System in einer Weise ermöglichen, die dauerhaft und stabil ist, einen leichten und raschen Wechsel der Novalung ^{®} ermöglicht und den Patienten in physischen Aktivitäten möglichst wenig behindert.

Erfindungsgemäß wird die generelle Aufgabe der Kanülierung mit annähernd 90°-igem Punktieren eines Gewebeabschnittes und insbesondere das Setzen einer Kanüle durch das Septum des Herzens dadurch gelöst, dass der Spitzenabschnitt elastisch und in Funktionslage bogenförmig in einem Winkel vom Hauptabschnitt abstehend ausgebildet ist, und dass der Spitzenabschnitt in Applikationsanlage durch eine Führungsvorrichtung in Richtung der Längserstreckung des Hauptabschnittes gehalten ist.

Weitere Merkmale der Erfindung sind unter anderem die folgenden:
Die Führungsvorrichtung weist bevorzugt einen Führungsdraht, eine über den Führungsdraht schiebbare erste Dilatationskanüle, eine über die erste Dilatationskanüle schiebbare zweite Dilatationskanüle und eine über der zweiten Dilatationskanüle angeordnete Führungshülse auf.

Ein weiteres bevorzugtes Merkmal besteht darin, dass die erste und zweite Dilatationskanüle in ihren Spitzenabschnitten in gleicher Weise wie der Spitzenabschnitt der Kanüle elastisch bogenförmig von den jeweiligen Hauptabschnitten in einem Winkel abstehend ausgebildet sind, wobei die erste Dilatationskanüle durch den Führungsdraht und die zweite Dilatationskanüle durch die in ihr angeordnete erste Dilatationskanüle geführt und entlang der Längserstreckung des Führungsdrahtes geführt ist.

Weiters ist es vorteilhaft, wenn die Führungshülse bei der Applikation vom Spitzenabschnitt entfernbar ist, sodass dieser die bogenförmige Krümmung einnehmen kann, und die Führungshülse zur Gänze entfernbar und durch die Kanüle ersetzbar ist, wonach der Führungsdraht und die ersten und zweiten Dilatationskanülen aus der Kanüle entfernbar sind, sodass der gesamte Innenquerschnitt der Kanüle freigegeben ist.

Weiters kann die Erfindung dadurch gekennzeichnet sein, dass der Spitzenabschnitt der zweiten Dilatationskanüle benachbart zur Spitze einen Dilatationsballon aufweist, dessen Durchmesser im aufgeblasenen Zustand dem Außendurchmesser der Kanüle entspricht und dessen Durchmesser im entspannten Zustand gleich oder kleiner als der Innendurchmesser der Kanüle ist.

Die Kanüle kann im Spitzenabschnitt eine die Kanüle fixierende Arretiervorrichtung, bevorzugt einen Ballon, aufweisen.

Die erste und zweite Dilatationskanüle und die Kanüle ist bevorzugt im Spitzenabschnitt in ungestütztem Zustand um einen Winkel von 20° bis 100°, bevorzugt 60° bis 95°, besonders bevorzugt 90°, gebogen.

In bevorzugter Weise ist die Anordnung zur transseptalen Kanülierung des linken Vorhofes des Herzens mit cervicalem Zugang zur transatrialen Punktion ausgebildet.

Die Oberflächen der Teile der Anordnung sind bevorzugt antithrombotisch ausgerüstet, insbesondere heparinisiert.

Die Erfindung betrifft auch die Anwendung der Anordnung zur transseptalen Kanülierung des linken Vorhofs des menschlichen Herzens mit cervicalem Zugang und weiters ein Verfahren zum Setzen von Kanülen und zur extrakorporalen Unterstützung von Patienten mit pulmonaler Hypertonie, oder auch anderen Formen respiratorischer Insuffizienz wie in den Ansprüchen definiert ist.

Nachfolgend wird die Erfindung anhand der Zeichnungen beispielsweise näher erläutert.
Fig. 1 zeigt schematisch eine Kanülenanordnung gemäß Stand der Technik.
Fig. 2 bis 7 zeigen schematisch schrittweise den Aufbau und die Anwendung der erfindungsgemäßen Anordnung beim menschlichen Herzen für das Setzen der Strömungskanüle in den linken Vorhof des Herzens.
Fig. 8 zeigt eine andere schematische Herzdarstellung zur Erklärung des Weges eines zusätzlichen Pulmonaliskatheters und Fig. 9 die Anordnung einer Vorrichtung zur extrakorporalen Membranoxidation.

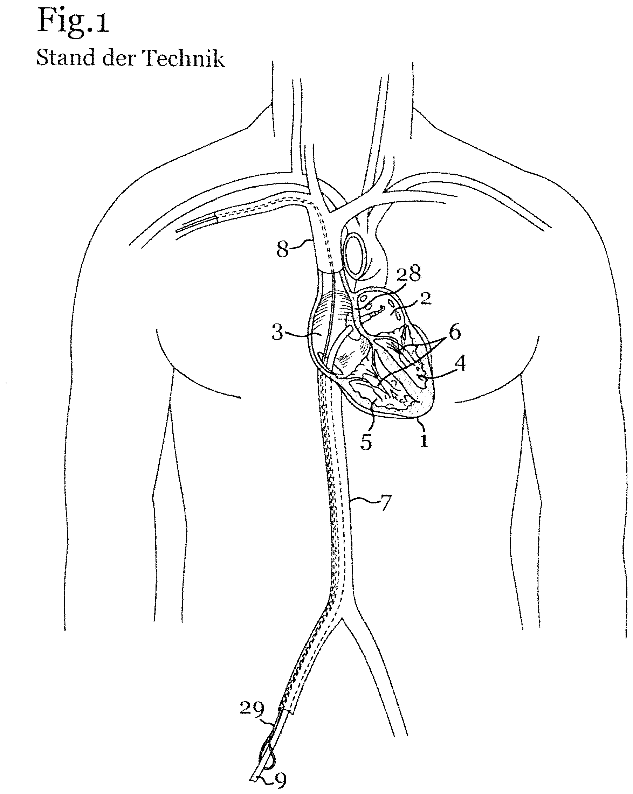

Die Fig. 1 zeigt den Stand der Technik, wie er beispielsweise durch die US 8 343 029 B2 geoffenbart wurde. Ein Katheter 28 wird bevorzugt durch einen Einschnitt in der Leistengegend durch die untere Hohlvene 7 nach oben in den rechten Vorhof 3 des Herzens 1 geschoben und durchdringt mit seinem Spitzenabschnitt das Septum 28, sodass der Katheter im linken Vorhof 2 endet. Diese Lage des Katheters und der damit eingebrachten Kanüle, die den Körper von der Leistengegend bis zum Herz durchragt, weist jedoch die eingangs beschriebenen Nachteile auf.

Die Fig. 1 zeigt als Stand der Technik auch eine eingelegte Schlaufe 29, die von der Vene im Bereich des Schlüsselbeins über die obere Hohlvene 8 nach unten geführt werden kann, um einen Führungsdraht, der in dem Katheter 9 vorgesehen ist, nach oben zu ziehen. Damit ist es aber nicht möglich eine Kanüle mit entsprechendem Volumen und einem Durchmesser von etwa 5 bis 9 mm bis in die linke Vorhofkammer zu applizieren.

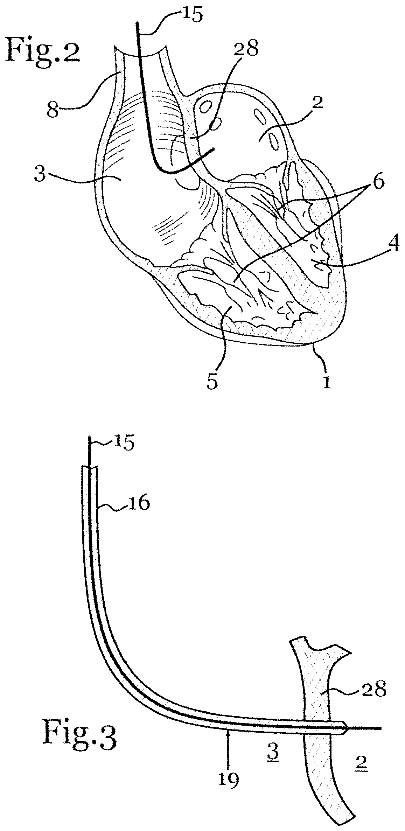

Der erfindungsgemäße Vorgang unter Anwendung der erfindungsgemäßen Kanülenanordnung wird anhand der Figuren 2 bis 7 erläutert.

Als erster Schritt wird gemäß Fig. 1 in bekannter Weise mittels des Katheters 9 der Führungsdraht 15 mit seiner Punktionsspitze durch das Septum 28 hindurch in den linken Vorhof 2 gebracht. Sodann wird der Führungsdraht - nach Entfernung des Katheters 9 - mit der Schlaufe 29 nach oben in die Halsvene oder die Schlüsselbeinvene gezogen, wobei das Ende des Führungsdrahtes durch eine Öffnung der Vene aus dem Körper herausgeführt ist.

In Fig. 2 ist gezeigt, wie der Führungsdraht 15 von der oberen Hohlvene 8 bis zum linken Vorhof 2 geführt ist. Dieser Führungsdraht - wobei das Wort "Draht" keine Einschränkung auf das Material darstellt - kann sehr weich und schmiegsam sein.

Falls sich herausstellt, dass der Führungsdraht 15 zu weich ist, um die erste Dilatationskanüle 16 angemessen zu führen, kann der Führungsdraht dadurch gegen einen steiferen Führungsdraht ausgetauscht werden, indem ein weiches Rohr, auch sheet genannt, über den weichen Draht bis in das Septum geschoben, der weiche Draht entfernt und der steife Draht als neuer Führungsdraht 24 bis zum linken Vorhof eingeschoben wird.

Ein steifer Draht kann aber auch später eingezogen werden, wenn die folgenden Dilatationskanülen schon gesetzt sind.

Gemäß Fig. 3 wird als nächster Schritt die erste Dilatationskanüle 16 über den Führungsdraht 15 oder 24 eingeschoben und die erste Dilatationskanüle 16 weist einen Spitzenabschnitt 19 auf, der mit ausreichender Spannung vorgebogen ist (jedoch nur so stark, dass er noch vom Führungsdraht gestreckt werden kann), sodass die erste Dilatationskanüle in etwa 90°-igem Winkel gegen das Septum 28 zu liegen kommt und durch das Septum 28 durchgestoßen werden kann. Damit ist ein erster noch relativ weicher und enger Zugang von oben zur linken Vorhofkammer des Herzens geschaffen.

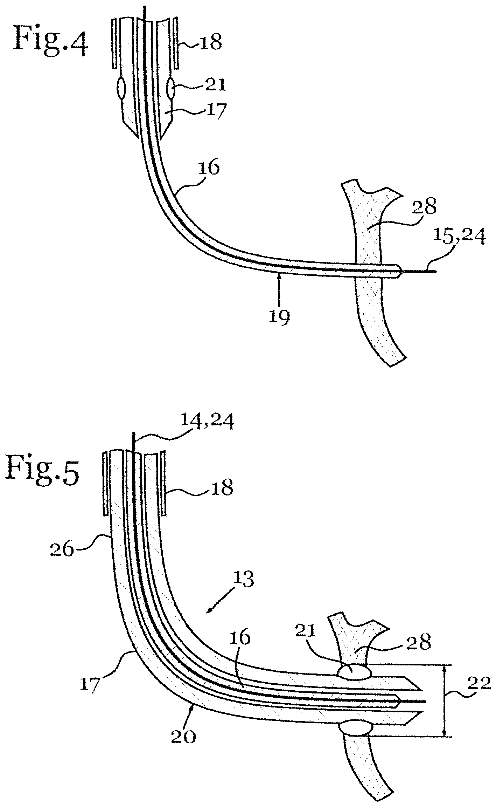

Gemäß Fig. 4 wird im nächsten Schritt über und entlang der ersten Dilatationskanüle 16 eine zweite Dilatationskanüle 17 geschoben. Auch die zweite Dilatationskanüle 17 ist im Spitzenabschnitt 20 mit genügender Vorspannung um etwa 90° gegenüber deren Hauptabschnitt 26 vorgebogen. Mit Hilfe der außen angeordneten Führungshülse 18 wird jedoch der Spitzenabschnitt 20 der zweiten Dilatationskanüle derart gerade gehalten, dass die Kanüle über den gesamten Hauptabschnitt der ersten Dilatationskanüle 16 gerade vorgeschoben werden kann. Zur Versteifung der zweiten Dilatationskanüle trägt auch die erste Dilatationskanüle ihren Beitrag bei. Sobald die zweite Dilatationskanüle in die Stellung gemäß Fig. 4 kommt, wird die Führungshülse 18 vom Arzt daran gehindert, die Vorschubbewegung der zweiten Dilatationskanüle mitzumachen, sodass der Spitzenabschnitt die Führungshülse 18 verlässt und entsprechend der Vorbiegung die gebogene Stellung einnimmt, wie in Fig. 5 dargestellt ist. Die ungestützte Lage der Kanülen gemäß Fig. 5 wird hier als Funktionslage bezeichnet wohingegen die gestützte, gestreckte Lage der jeweiligen Spitzenabschnitte als Applikationslage bezeichnet wird.

Wie in Fig. 5 ebenfalls dargestellt ist, wird auch die zweite Dilatationskanüle 17 bis in den linken Vorhof 2 durch das Septum 28 geschoben, wobei im Spitzenabschnitt der zweiten Dilatationskanüle bevorzugt ein Dilatationsballon 21 vorgesehen ist, der in bekannter Weise aufgeblasen werden kann, um das Loch im Septum 28 für die endgültig einzuführende Kanüle zu erweitern.

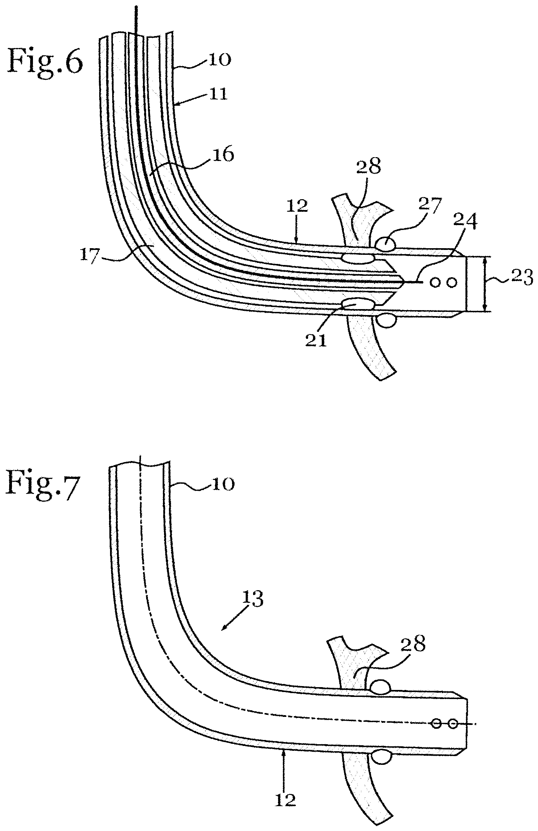

Als nächster Schritt wird die Führungshülse 18 von den Dilatationskanülen nach außen abgezogen und es wird die letztendlich benötigte Kanüle 10 eingeschoben, die ebenfalls einen vorgebogenen Spitzenabschnitt aufweisen kann, wobei während der Applikation beim Einschieben über die beiden vorher eingebrachten Dilatationskanülen die notwendige Steifheit gegeben ist, sodass die Vorbiegung überwunden wird und die Kanüle 10 in gerader Form eingeschoben werden kann. Sobald die Biegung gemäß Fig. 6 erreicht ist, kann die Kanüle 10 der Biegung leicht folgen und durch das Septum hindurch in den linken Vorhof eingeschoben werden.

Zur Arretierung der Kanüle ist ein Arretierballon eingezeichnet, der als Arretiervorrichtung 27 ballonförmig ist. Die Arretierung kann aber auch durch andere Mittel erfolgen, wie z.B. Verspreitzungen.

Wie in Fig. 6 ebenfalls zu sehen ist, kann der Dilatationsballon 21 der zweiten Dilatationskanüle 17 durch Auslassen des Luftdrucks derart entspannt werden, dass der Außendurchmesser im Ballonbereich etwa dem Innendurchmesser der Kanüle 10 entspricht. Damit wird der Kanüle 10 der Weg durch das Septum freigegeben. Im aufgeblasenem Zustand gemäß Fig. 5 weist der Ballon 21 einen Außendurchmesser auf, der dem Außendurchmesser der Kanüle 10 entspricht und somit das Loch im Septum derart ausweitet, dass die Kanüle 10 leicht eingeschoben werden kann.

Die Fig. 7 zeigt letztendlich die fertige Stellung der Kanüle 10, aus der alle Innenteile nämlich Führungsdraht 15 oder 24 und beide Dilatationskanülen 16, 17 herausgezogen worden sind. Somit steht das volle Querschnittsvolumen der Strömungskanüle 10 als Linke-Vorhof-Kanüle (LA-Kanüle) zur Verfügung, um die gewünschten Durchflussmengen und Strömungsbedingungen herzustellen.

Die Zahl der übereinander zu schiebenden Dilatationskanülen ist nicht auf zwei beschränkt. Wenn der Dilatationsballon 21 vermieden werden soll, kann auch noch eine dritte oder vierte Kanüle mit den oben genannten Eigenschaften übergezogen werden, um die notwendige Erweiterung des Loches im Septum und den erforderlichen Strömungsquerschnitt in der letzten Endes eingesetzten Strömungskanüle 10 zu erreichen. Die Dilatationskanülen weisen bevorzugt einen inneren freien Querschnitt auf, der das Schieben der Kanüle über den innenliegenden Führungsdraht oder über die innenliegende Dilatationskanüle erlaubt.

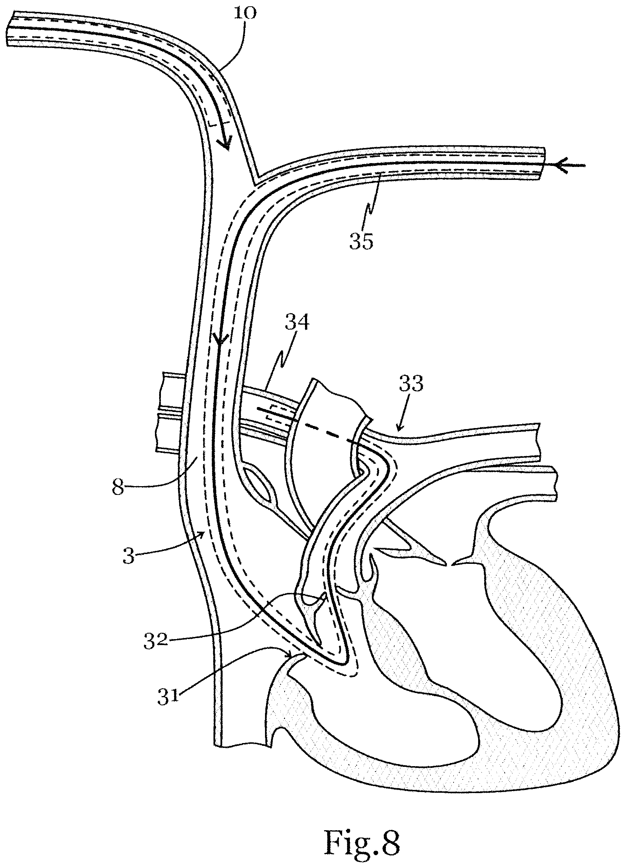

Die Fig. 8 ist eine schematische Darstellung der Anordnung einer weiteren Strömungskanüle als Pulmonaliskanüle 35 für die Kanülierung der Pulmonalisaterie 33. Dabei wird über die obere Hohlvene 8 ein herkömmlicher Pulmonaliskatheter eingesetzt, der relativ weich ist und einen Ballon trägt. Ein solcher Pulmonaliskatheter kann mit einem Führungsdraht ausgestattet werden, sodass der Führungsdraht mit dem Katheter durch den natürlichen Blutstrom über den rechten Vorhof 3, durch die Trikuspidalklappe 31 in die rechte Herzkammer und von dort durch die Pulmonalklappe 32 in die Pulmonalarterie 33 geschwemmt wird. Bevorzugt soll der Führungsdraht in die rechte Pulmonalarterie 34 eingesetzt werden.

Sobald der Führungsdraht seine gewünschte Lage angenommen hat, können in der bisher beschriebenen Weise schrittweise aufeinanderfolgend eine oder mehrere Kanülen eingeschoben werden, wobei durch den Führungsdraht die Führung übernommen wird und die Kanülen durch entsprechende Flexibilität die Biegung in die gewünschte Form mitmachen können.

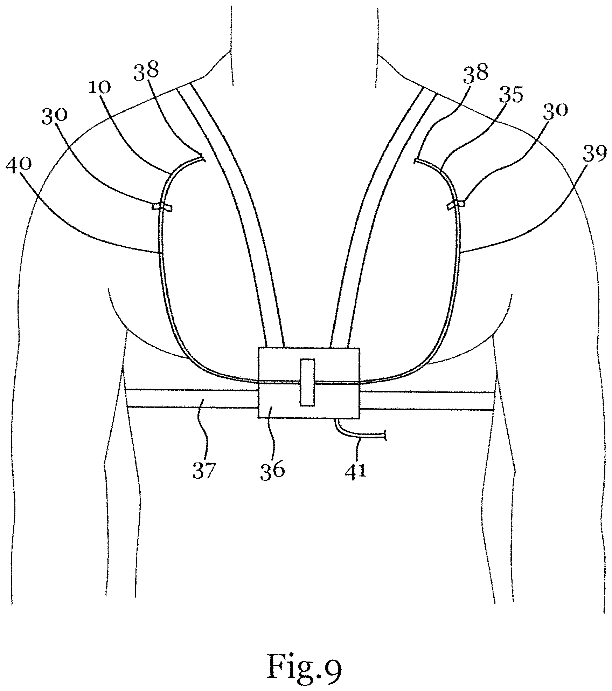

Mithilfe der gemäß Fig. 2 bis 7 in den linken Vorhof eingeführten ersten Strömungskanüle 10 (LA-Kanüle) und der gemäß Fig. 8 in die Pulmonalarterie eingeführten Pulmonaliskanüle 35 ist es möglich, Blut aus der Pulmonalarterie in den linken Vorhof abzuleiten, wobei über entsprechende Schlauchverbindungen außerhalb des Körpers eine tragbare Vorrichtung angeschlossen werden kann, in der aus dem Blut CO2 entfernt und 02 zugeführt wird. Dies ist in Fig. 9 dargestellt.

In Fig. 8 ist die zur linken Vorhofkammer führende Strömungskanüle 10 (LA-Kanüle) nur im oberen Abschnitt angedeutet. Die vollständige Lage ist den Fig. 2 bis 7 zu entnehmen.

Alternativ kann anstelle der Blutentnahme aus der Pulmonalarterie eine venöse Blutentnahme erfolgen, beispielsweise aus der oberen Hohlvene. Mithilfe einer Pumpenunterstützung (insbesondere einer Kreiselpumpe) kann das venöse Blut über die Novalung oder über ein vergleichbares Gerät und die LA-Kanüle in den linken Vorhof des Herzens zurückgeführt werden. Diese Alternative ist für jene Fälle bevorzugt, wenn die pulmonale Hypertonie nicht übermäßig stark ausgeprägt ist und die Lungenfunktion ersetzt oder unterstützt werden soll.

Die Vorgangsweise und weitere Ausführungen werden im Folgenden beschrieben:
Lungenhochdruck im Endstadium führt zum rechtsventrikulären Versagen. Wenn die medikamentöse Therapie ausgeschöpft ist, kann dieses Versagen nur durch eine mechanische Kreislaufunterstützung verhindert werden. Alle bisherigen Verfahren sind entweder operativ zu installieren, oder nur für eine kurzzeitige Anwendung ohne Möglichkeit für Mobilität des Patienten geeignet.

Die perkutane Einbringung von Kanülen über einen Zugang von der oberen Körperhälfte gemäß Erfindung ermöglicht es, einen dauerhaften Zugang herzustellen. In Verbindung mit einer Membran, die CO2 entfernt und 02 zuführt (z.B. Novalung, ILA = interventioneller Lungassist) kann man Blut von der Pulmonalarterie in den linken Vorhof umleiten. Dies ermöglicht es, eine Druckreduzierung in der Pulmonalarterie zu erreichen, und gleichzeitig die Sauerstoffsättigung des arteriellen Blutes aufrecht zu erhalten. Der Blutfluss erfolgt dabei rein passiv, getrieben durch die hohe Druckeffizienz zwischen Pulmonalarterie und linken Vorhof, oder wenn dies nicht ausreichend ist, durch einen zusätzlichen Pumpenantrieb, wie zum Beispiel mittels Kreiselpumpe.

Durch die erfindungsgemäße entsprechende Gestaltung der Kanülen in Verbindung mit einer Tragtaschenkonstruktion kann eine eingeschränkte Mobilität der Patienten erzielt werden.

Um dieses Konzept zu verwirklichen bedarf es:
- einer geeigneten Kanüle zum Ableiten des Blutes aus der Pulmonalarterie, nämlich der Pulmonaliskanüle 35,
- einer geeignet Konnektionsmöglichkeit dieser Kanüle mit einem Konnektor 30, wie er bekannt ist,
- einer Novalung-Membran 36 (mit oder ohne Pumpunterstützung) oder eines vergleichbaren Gerätes,
- einer geeignet Konnektionsmöglichkeit 30 der Novalung mit der
- Kanüle zum Rückführen des Blutes in den linken Vorhof, nämlich der LA-Kanüle 10.

Weiters ist die Tragtaschenkonstruktion 37 vorgesehen.

Die Pulmonaliskanüle wird durch perkutane Punktion der Vena Subclavia, bevorzugt der linken Vena Subclavia eingebracht. Folgende Schritte sind dazu notwendig:
- Punktion der Vene an der Cision 38
- Einbringen eines ersten weichen Führungsdrahtes
- Darüber Einbringen eines Katheters mit Ballon an der Spitze. Diesen ist ähnlich wie einem herkömmlichen Pulmonaliskatheter, aber geeignet um als Führung für einen darin einzubringenden steiferen Führungsdraht zu dienen. Dieser Katheter wird durch Aufblasen des Ballons passiv durch die obere Hohlvene, die Trikuspidalklappe 31, die Pulmonalklappe 32 in die Pulmonalarterie durch den Blutfluss eingeschwemmt. Danach wird ein zweiter steifer Führungsdraht innerhalb des Ballonkatheters in die Pulmonalarterie vorgeschoben und der Ballonkatheter entfernt.
- Nunmehr wird die eigentliche Pulmonaliskanüle über den in der Pulmonalarterie liegenden Führungsdraht eingebracht, und dort positioniert. Danach wird der Führungsdraht entfernt. Wenn notwendig kann als Zwischenschritt noch eine Führungskanüle mit erweitertem Querschnitt eingesetzt werden.

Die Pulmonaliskanüle 35 hat an ihrem proximalen Ende eine Konnektionsvorrichtung 30, die eine rasche, einfache und stufenlose Verbindung mit der Novalung ermöglicht. Dies geschieht durch eine geeignete erste Konnektionskanüle 39, sodass die notwendige Konfiguration erreicht wird, um einen direkten und ungehinderten Zugang zur Novalung zu ermöglichen.

Die verwendete Novalung entspricht den herkömmlich verwendeten Membranvorrichtungen. Diese kann in einer geeigneten Tragevorrichtung 37 so vor der Brust des Patienten montiert werden, dass dieser zwar eingeschränkt aber doch mobil ist; siehe dazu Fig. 9. Die notwendige Sauerstoffzufuhr erfolgt durch einen Sauerstoffschlauch 41 aus einem separaten Sauerstoffreservoir (nicht dargestellt). Im Falle der Verwendung einer ILA active mit einem Pumpsystem ist die Anbringung der ganzen Vorrichtung auf einer Tragekonsole möglich. Dementsprechend sind die Konnektoren in einer anderen dafür geeigneten Konfiguration zu gestalten, was jedem Techniker der Medizintechnik geläufig ist.

In ähnlicher Weise ist eine zweite Konnektionskanüle 40 erforderlich, die die notwendige Verbindung zwischen Novalung 36 und Linksvorhofkanüle (LA-Kanüle 10) ermöglicht.

Die Linksvorhofkanüle wird entsprechend den Fig. 2 bis Fig. 7 eingebracht, die dann oberhalb durch die obere Hohlvene 8 in das Herz bis zum linken Vorhof geführt ist.

Diese Anordnung kann für einen temporären Einsatz, zum Beispiel als Überbrückung bis zu einer Transplantation, oder einer Verbesserung des Gesamtzustandes durch allgemeine Erholung des Patienten, eventuell auch nach Operationen verwendet werden. Sie kann aber auch als permanente Unterstützung bei Patienten dienen, die keine Aussicht auf Verbesserung haben und keine Kandidaten für eine Transplantation darstellen.

### Bezugszeichenliste

- 1.: Herz
- 2.: Linker Vorhof
- 3.: Rechter Vorhof
- 4.: Linke Kammer
- 5.: Rechte Kammer
- 6.: Herzklappen
- 7.: Untere Hohlvene
- 8.: Obere Hohlvene
- 9.: Katheter
- 10.: Strömungskanüle (LA-Kanüle)
- 11.: Hauptabschnitt Kanüle
- 12.: Spitzenabschnitt Kanüle
- 13.: Winkel
- 14.: Führungsvorrichtung
- 15.: Führungsdraht weich
- 16.: Erste Dilatationskanüle
- 17.: Zweite Dilatationskanüle
- 18.: Führungshülse
- 19.: Spitzenabschnitt erster Dilatationskanüle
- 20.: Spitzenabschnitt zweite Dilatationskanüle
- 21.: Dilatationsballon
- 22.: Durchmesser Dilatationsballon aufgeblasen
- 23.: Durchmesser Dilatationsballon entspannt
- 24.: Führungsdraht steif
- 25.: Hauptabschnitt erste Dilatationskanüle
- 26.: Hauptabschnitt zweite Dilatationskanüle
- 27.: Arretiervorrichtung
- 28.: Septum
- 29.: Schlaufe
- 30.: Konnektor
- 31.: Trikuspidalklappe
- 32.: Pulmonalklappe
- 33.: Pulmonalarterie
- 34.: Rechte Pulmonalarterie
- 35.: Pulmonaliskanüle
- 36.: Novalung-Membran
- 37.: Tragegestell
- 38.: Cision
- 39.: Konnektionskanüle
- 40.: Konnektionskanüle
- 41.: Sauerstoffschlauch

## Patentansprüche

1. Kanülenanordnung zum Applizieren einer Strömungskanüle zum Ein-, Durch- oder Umleiten von Strömungsmedien, insbesondere Blut, im menschlichen oder tierischen Körper, wobei die Strömungskanüle (10) einen Hauptabschnitt (11) und einen Spitzenabschnitt (12) aufweist, **dadurch gekennzeichnet, dass** der Spitzenabschnitt (12) elastisch und in Funktionslage bogenförmig in einem Winkel vom Hauptabschnitt (11) abstehend ausgebildet ist, und dass der Spitzenabschnitt (12) in Applikationslage durch eine Führungsvorrichtung (14) in Richtung der Längserstreckung des Hauptabschnittes (11) gehalten ist.

2. Anordnung nach Anspruch 1, **dadurch gekennzeichnet, dass** die Führungsvorrichtung (4)
- einen Führungsdraht (15, 24, 30),
- eine über den Führungsdraht schiebbare erste Dilatationskanüle (16),
- eine über die erste Dilatationskanüle (10) schiebbare zweite Dilatationskanüle (17) und gegebenenfalls noch weiterer Dilatationskanülen und
- eine über der zweiten oder äußersten Dilatationskanüle angeordnete Führungshülse (18) umfasst.

3. Anordnung nach Anspruch 2, **dadurch gekennzeichnet, dass** die erste und zweite und gegebenenfalls jede weitere Dilatationskanüle (16, 17) in ihren Spitzenabschnitten (19, 20) in gleicher Weise wie der Spitzenabschnitt (12) der Strömungskanüle (10) elastisch bogenförmig von den jeweiligen Hauptabschnitten (11, 25, 26) in einem Winkel abstehend ausgebildet sind, wobei die erste Dilatationskanüle (16) durch den Führungsdraht (15, 24) und die zweite Dilatationskanüle (17) und gegebenenfalls jede weitere durch die in ihr angeordnete erste Dilatationskanüle (16) geführt und entlang der Längserstreckung des Führungsdrahtes (24) geführt ist.

4. Anordnung nach einem der Ansprüche 1 bis 3, **dadurch gekennzeichnet, dass** die Führungshülse (18) bei der Applikation vom Spitzenabschnitt (12) entfernbar ist, sodass dieser die bogenförmige Krümmung einnehmen kann, und dass die Führungshülse (18) zur Gänze entfernbar und durch die Strömungskanüle (10) ersetzbar ist, wonach der Führungsdraht (24) und die ersten und zweiten und etwaige weitere Dilatationskanülen (16, 17) auf der Strömungskanüle (10) entfernbar sind, sodass der gesamte Innenquerschnitt der Strömungskanüle (10) freigegeben ist.

5. Anordnung nach einem der Ansprüche 2 bis 4, **dadurch gekennzeichnet, dass** der Spitzenabschnitt (20) der zweiten oder äußersten Dilatationskanüle (17) benachbart zur Spitze einen Dilatationsballon (21) aufweist, dessen Durchmesser (22) im aufgeblasenem Zustand den Außendurchmesser der Kanüle und dessen Durchmesser (23) im entspannten Zustand kleiner als der oder gleich dem Innendurchmesser der Kanüle ist.

6. Anordnung nach einem der Ansprüche 1 bis 5, **dadurch gekennzeichnet, dass** die Strömungskanüle (10) im Spitzenabschnitt (12) eine die Kanüle (10) fixierende Arretiervorrichtung (27), bevorzugt einen Ballon, aufweist.

7. Anordnung nach einem der Ansprüche 1 bis 6, **dadurch gekennzeichnet, dass** die erste, zweite und eventuell folgende Dilatationskanülen (16, 17) und die Strömungskanüle (10) im Spitzenabschnitt (12) in ungestütztem Zustand um einen Winkel von 20° bis 100°, bevorzugt 60° bis 95°, besonders bevorzugt 90°, gebogen ist.

8. Anordnung nach einem der Ansprüche 1 bis 7, **dadurch gekennzeichnet, dass** sie zur transseptalen Kanülierung des linken Vorhofes (2) des Herzens (1) mit Zugang über die obere Hohlvene zur transatrialen Punktion ausgebildet ist.

9. Anordnung nach einem der Ansprüche 1 bis 8, **dadurch gekennzeichnet, dass** die Oberflächen der Teile der Anordnung antithrombotisch gerüstet, insbesondere heparinisert, sind.

10. Anwendung der Anordnung nach einem der Ansprüche 1 bis 9 zur transseptalen Kanülierung des linken Vorhofs des menschlichen Herzens mit cervicalem Zugang.

11. Verfahren zum Setzen einer Kanüle zum Ein-, Durch- oder Umleiten von Strömungsmedien, insbesondere Blut, im menschlichen oder tierischen Körper, insbesondere bei der Indikation Pulmonale Hypertonie, **dadurch gekennzeichnet,**
- **dass** eine Kanülenanordnung mit einer Strömungskanüle vorgesehen wird, die einen Hauptabschnitt und einen Spitzenabschnitt aufweist, wobei der Spitzenabschnitt elastisch und in Funktionslage bogenförmig in einem Winkel vom Hauptabschnitt abstehend ausgebildet ist, und der Spitzenabschnitt in Applikationslage durch eine Führungsvorrichtung in Richtung der Längserstreckung des Hauptabschnittes gehalten ist,
- **dass** ein Katheter mit einem innen geführten Führungsdraht vorgesehen wird,
- **dass** durch eine Incision an der Halsvene oder Schlüsselbeinvene eine Fangschlaufe bis zur Vena Femoralis (Oberschenkelvene) eingesetzt wird,
- **dass** in der Leistengegend an der Vena Femoralis der Katheter mit dem Führungsdraht bis zur rechten Herzkammer geführt und der Führungsdraht durch das Septum gestoßen und dort verankert wird,
- **dass** der Katheter entfernt wird,
- **dass** mit der Fangschlaufe der Führungsdraht zur Halsvene oder Schlüsselbeinvene hochgezogen wird,
- und sodann über den Führungsdraht von der Halsvene oder Schlüsselbeinvene die Kanülenanordnung gesetzt wird, und
- **dass** nach Setzen der Strömungskanüle die innenliegenden Dilatationskanülen und der Führungsdraht entfernt werden.

12. Verfahren zur extrakorporalen Unterstützung von Patienten mit pulmonaler Hypertension, wobei
- gemäß Anspruch 11 eine Strömungskanüle als linke Vorhofkanüle zum Durchleiten von Blut in die linke Vorhofkammer des Herzens,
- eine Strömungskanüle als Pulmonaliskanüle zur Ableitung von Blut aus der Pulmonalarterie,
- eine Novalung-Membran oder ein ähnliches Gerät mit der zugehörigen Sauerstoffversorgung, eventuell mit Pumpunterstützung und die Verbindungskanülen
vorgesehen sind.

13. Verfahren nach Anspruch 12, **dadurch gekennzeichnet, dass** die linke Vorhofkanüle durch die Incision aus der rechten Schlüsselbeinvene und die Pulmonaliskanüle aus der linken Schlüsselbeinvene durch Incision aus dem Körper herausgeführt und über die Verbindungskanülen mit der Novalung-Membran verbunden werden.

14. Verfahren zur extrakorporalen Unterstützung von Patienten mit Lungeninsuffizienz, wobei
- gemäß Anspruch 11 eine Strömungskanüle als linke Vorhofkanüle zum Durchleiten von Blut in die linke Vorhofkammer des Herzens und
- eine Kanüle zur venösen Blutentnahme, beispielsweise in die obere Hohlvene gesetzt werden
- und die Kanülen mit der Novalung oder vergleichbaren Gerät und gegebenenfalls einer extrakorporalen Pumpe, bevorzugt Kreiselpumpe, verbunden werden, um die Lungenfunktion zu ersetzen oder zu unterstützen.
